# DEMANDE DE BREVET EUROPEEN

(11) **EP 1 525 877 A1**
(43) Date de publication de la demande: **27.04.2005**
(21) Numéro de dépôt: 04292390.4
(22) Date de dépôt: 08.10.2004
(51) Int. Cl.: A61K 7/11

(54) **Compositon cosmètique capillaire à base de polymères fixants et de composés susceptible de gonfler sous l'action de la chaleur.**

(30) Priorité: 24.10.2003 FR 0312486
(71) Demandeur: L'OREAL, 75008 Paris (FR)
(72) Inventeur: Rollat, Isabelle, 75017 Paris (FR); Gawtrey, Jonathan, 92100 Boulogne (FR); Condamine, Christiane, 95610 Eragny s/Orge (FR)
(74) Mandataire: Casalonga, Axel

(57) **Abrégé**

L'invention concerne une composition cosmétique capillaire, caractérisée en ce qu'elle comprend, dans un milieu cosmétiquement acceptable, au moins un polymère fixant et au moins un composé susceptible de gonfler sous l'action de la chaleur. Elle a également pour objet un procédé de fixation et/ou de maintien de la coiffure.

## Description

La présente invention est relative à de nouvelles compositions cosmétiques capillaires à base de polymères fixants et de composés susceptibles de gonfler sous l'action de la chaleur, et notamment de microsphères à enveloppe thermoplastique. Elle concerne également un procédé cosmétique, en particulier un procédé de fixation et/ou de maintien de la coiffure, ainsi que l'utilisation en cosmétique de ces compositions.

Les produits capillaires destinés à la mise en forme et au maintien de la coiffure comprennent généralement un ou plusieurs polymères fixants en solution alcoolique ou aqueuse. Les polymères fixants assurent la formation de soudures entre les cheveux. Les polymères fixants généralement employés sont des polymères filmogènes solubles ou dispersibles dans l'eau et dans les alcools tels que les copolymères d'acétate de vinyle et d'acide crotonique, les résines acryliques anioniques ou amphotères, les polyuréthanes.

Les soudures entre les cheveux doivent être suffisamment résistantes pour assurer le maintien des cheveux. Elles doivent également être suffisamment fragiles pour que l'utilisateur puisse, en peignant ou en brossant les cheveux, rompre les soudures sans heurter le cuir chevelu ni endommager les cheveux, ce qui n'est pas forcément le cas des compositions de maintien et/ou de fixation des cheveux connues.

Il est ainsi nécessaire de disposer de produits capillaires qui puissent assurer un maintien satisfaisant des cheveux, tout en permettant à l'utilisateur de pouvoir ensuite se coiffer sans heurter le cuir chevelu ni endommager les cheveux.

La présente invention se propose de fournir une solution à cette attente.

En particulier, la Demanderesse vient de découvrir de façon surprenante qu'une composition cosmétique capillaire comportant, dans un milieu cosmétiquement acceptable, au moins un polymère fixant et au moins un composé susceptible de gonfler sous l'action de la chaleur, conférait aux cheveux une fixation satisfaisante et durable et que, sous l'effet de la chaleur, les cheveux étaient ensuite faciles à démêler.

Elle a en effet constaté que, sous l'action de la chaleur, l'expansion du composé susceptible de gonfler entraînait une fragilisation des soudures entre les cheveux, conduisant ainsi à l'amélioration nette du démêlage des cheveux.

D'autres objets de l'invention apparaîtront à la lecture de la description et des exemples qui suivent.

La présente invention a donc pour objet une composition cosmétique capillaire, caractérisée en ce qu'elle comprend, dans un milieu cosmétiquement acceptable, au moins un polymère fixant et au moins un composé susceptible de gonfler sous l'action de la chaleur.

Un autre objet de l'invention concerne un procédé cosmétique capillaire, en particulier un procédé de fixation et/ou de maintien de la coiffure mettant en oeuvre les compositions selon l'invention.

L'invention a également pour objet l'utilisation cosmétique des compositions selon l'invention.

Au sens de la présente invention, on entend par polymère fixant tout polymère permettant de donner une forme à la chevelure ou de maintenir cette forme.

Les polymères fixants convenant dans l'invention sont notamment choisis parmi les polymères fixants cationiques, anioniques, amphotères, non ioniques et leurs mélanges.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 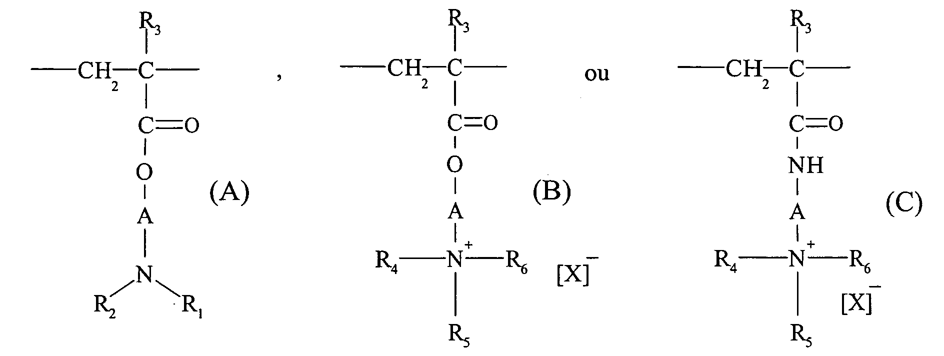 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammomium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT®" par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français Nos 2 077 143 et 2 393 573,
   - les polymères à chaîne grasse et à motif vinylpyrrolidone, tels que les produit vendus sous la dénomination Stylèze W20 et Stylèze W10 par la société ISP,
   - les terpolymères méthacrylate de diméthylaminoéthyle/vinylcaprolactame/vinylpyrrolidone tels que le produit commercialisé sous la dénomination GAFFIX® VC 713 par la société ISP, et
   - les copolymères vinylpyrrolidone/méthacrylamide de diméthylaminopropyle quaternisé tels que notamment le produit commercialisé sous la dénomination "GAFQUAT® HS 100" par la société ISP ;
(2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL;
(3) les copolymères quaternaires de vinylpyrrolidone et de vinylimidazole ;
(4) les chitosanes ou leurs sels ; les sels utilisables sont en particulier l'acétate, le lactate, le glutamate, le gluconate ou le pyrrolidone-carboxylate de chitosane.
   Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 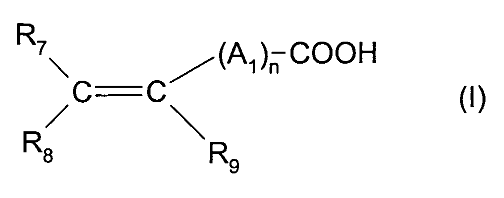 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n°^{s} 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en Ci-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les brevets français n^{os} 2 350 384 et 2 357 241 de la demanderesse.
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan® 500 et Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

Comme autre polymère fixant anionique utilisable selon l'invention, on peut citer le polymère anionique séquencé branché vendu sous la dénomination FIXATE G100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination FIXATE G100 par la société NOVEON.

Parmi les polymères fixants anioniques cités ci-dessus, on préfère plus particulièrement utiliser dans le cadre de la présente invention les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination FIXATE G100 par la société NOVEON.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale : 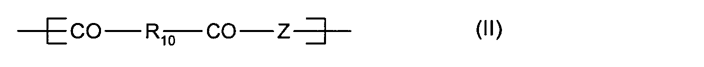 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 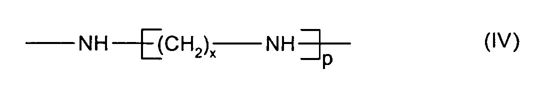 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 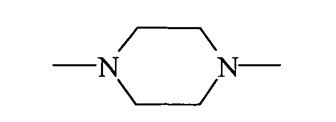
   c) dans les proportions de 0 à 20 % en moles, le groupe -NH-(CH₂)₆-NH- dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 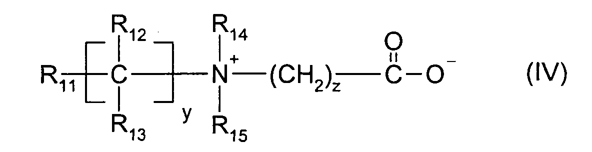 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tels que le produit vendu sous la dénomination DIAFORMER Z301 par la société SANDOZ.
(5) les polymères dérivés du chitosane comportant des motifs monomères répondant aux formules suivantes : 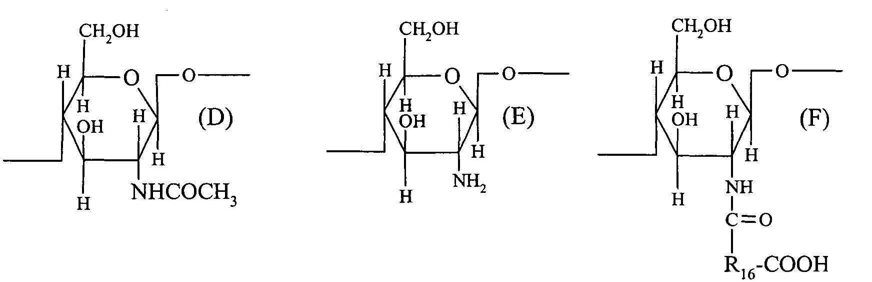 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 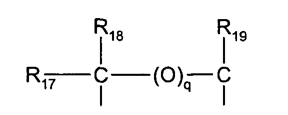 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 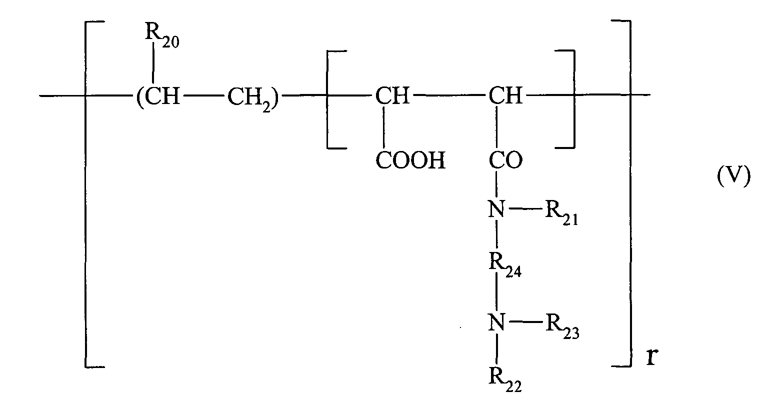 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 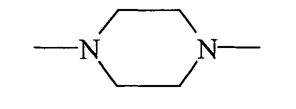 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')
   où D désigne un groupe 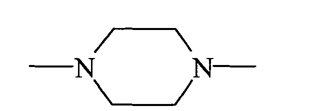 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères décrits ci-dessus, les plus particulièrement préférés selon l'invention sont ceux de la famille (3) tels que les copolymères dont la dénomination CTFA est Octylacrylamide/ acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthyl-méthacrylate de méthyle vendu par exemple sous la dénomination DIAFORMER® Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyle des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 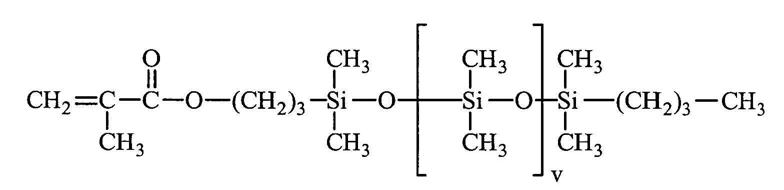
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex® Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR® et LUVISET® Si PUR par la société BASF.

Dans les compositions de l'invention, la teneur en polymère(s) fixant(s) est généralement comprise entre 0,1 et 20 %, et de préférence entre 0,2 et 15 %, et encore plus préférentiellement entre 0,5 et 10 % en poids du poids total de la composition.

Comme indiqué précédemment, les compositions selon l'invention comprennent au moins un composé susceptible de gonfler sous l'action de la chaleur.

Il peut s'agir notamment d'un composé qui réagit, sous l'action de la chaleur, pour libérer un gaz qui se trouve emprisonné dans la matrice du dépôt.

Le composé susceptible de gonfler à la chaleur peut également se présenter sous la forme de particules thermoexpansibles.

Par l'expression « particules thermoexpansibles », on désigne plus particulièrement des particules susceptibles de se déformer et de s'expanser à la chaleur. Les particules au sens de la présente invention peuvent encore être des particules thermodéformables non expansées. Elles se distinguent à ce titre des particules expansées qui ne sont précisément plus sujettes à une déformation sous l'action de la chaleur, à l'image par exemple des particules de polyvinylidène/acrylonitrile commercialisées sous la dénomination générale d' «Expancel®» par la société AKZO NOBEL sous les références particulières «Expancel® WE » ou « DE ».

Les particules utilisées dans les compositions de l'invention sont capables de s'expanser sous l'action d'une température généralement supérieure ou égale à 45°C, notamment supérieure ou égale à 50°C, en particulier supérieure ou égale à 60°C, plus particulièrement supérieure ou égale à 70°C. En particulier, il peut s'agir d'une température supérieure ou égale à 80°C, en particulier supérieure ou égale à 85°C, plus particulièrement supérieure ou égale à 90°C et allant jusqu'à 190-200°C.

Avantageusement, ces particules ne sont pas sensibles à la présence d'eau.

En particulier les particules utilisées dans la présente invention peuvent être thermoplastiques. Par l'expression thermoplastique, on désigne des particules qui sont susceptibles de se déformer sous l'action de la chaleur et de conserver leur nouvelle forme, y compris après refroidissement, notamment à température ambiante.

Les particules utilisées dans la présente invention sont généralement des particules creuses comportant une enveloppe continue et au moins une cavité.

L'enveloppe des particules est de préférence flexible pour se prêter à une déformation mécanique. Elle comprend généralement au moins un polymère, homo- ou copolymère, formé à partir de monomères à insaturations éthyléniques. Des exemples de telles particules sont notamment décrits dans les documents EP-A-56219, EP-A-348 372, EP-A-486 080, EP-A-320 473, EP-A-112 807 et US-A-3 615 972.

Les monomères utilisés peuvent être en particulier des esters d'acide méthacrylique ou acrylique, tels que l'acrylate et le méthacrylate de méthyle, le chlorure de vinylidène, l'acrylonitrile, le styrène et ses dérivés, le butadiène et ses dérivés, et leurs mélanges.

A titre représentatif et non limitatif des polymères susceptibles de composer l'enveloppe des particules utilisées dans la présente invention, on peut notamment citer des polymères comportant au moins des motifs dérivés d'acrylate ou de méthacrylate de méthyle, des polymères comportant au moins des motifs dérivés d'acrylonitrile, des polymères comportant au moins des motifs dérivés d'acrylonitrile et de méthacrylate de méthyle, des polymères comportant au moins des motifs dérivés de styrène et d'acrylonitrile, des polymères comportant au moins des motifs dérivés de chlorure de vinylidène et d'acrylonitrile et des polymères comportant au moins des motifs dérivés de chlorure de chlorure de vinylidène et de chlorure de vinyle. En particulier, ledit polymère peut être choisi parmi les polymères de chlorure de vinylidène/acrylonitrile/méthacrylate de méthyle, les polymères d'acrylonitrile/méthacrylate de méthyle et les homopolymères d'acrylonitrile.

Les particules contiennent généralement au sein d'une ou plusieurs cavité(s) au moins un composé capable de manifester, en réponse à un chauffage à une température située dans une gamme allant de 45°C à 200°C et à pression sensiblement constante, une augmentation significative de son volume à température ambiante.

On entend par l'expression « augmentation significative de son volume », une augmentation d'au moins un facteur 30, en particulier d'au moins un facteur 40 et plus particulièrement d'au moins un facteur 50 du volume occupé.

De façon générale, le composé contenu à l'intérieur de la cavité peut être à température ambiante un composé gazeux ou bien un composé liquide présentant une température de vaporisation située dans la gamme de 45°C à 200°C, en particulier dans la gamme de 80°C à 200°C, et plus particulièrement supérieure ou égale à 100°C.

Selon une première variante, ce composé est à l'état gazeux dans la particule et se dilate sous l'effet de la chaleur. Parmi les composés à l'état gazeux, on peut citer l'air, l'azote, les hydrocarbures comprenant 1, 2, 3 ou 4 atomes de carbone comme en particulier le butane, l'isobutane et leurs mélanges.

Selon une deuxième variante, le composé contenu dans la cavité est un composé liquide tel que défini précédemment. Parmi ces composés, on peut citer les hydrocarbures, notamment ayant de 5 à 15, en particulier de 5 à 12 et plus particulièrement de 5 à 10 atomes de carbone. Il peut s'agir en particulier de d'un composé, choisi parmi le n-pentane, l'iso-pentane et le néo-pentane.

La température d'expansion de la particule dépend à la fois de la nature du composé présent dans sa cavité, et de celle du polymère formant son enveloppe, et peut en particulier aller de 45 à 200°C, et être notamment supérieure ou égale à 80°C et en particulier supérieure ou égale à 100°C.

Les particules utilisées dans les compositions selon l'invention peuvent être sèches ou hydratées.

Ces particules peuvent être de différentes formes. Elles peuvent être de forme globulaire, voire sphérique, ou peuvent également être allongées.

Selon un mode de réalisation particulier, les particules non expansées de l'invention sont sphériques et présentent une granulométrie, exprimée en diamètre « effectif » moyen en poids D[0,5], allant de 0,5 µm à 200 µm, notamment de 1 µm à 100 µm, en particulier de 4 µm à 50 µm et plus particulièrement de 5 µm à 40 µm.

Selon un mode de réalisation particulier, les particules utilisées dans les compositions selon l'invention ont une forme de fibre. Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150. Les fibres présentent en particulier une longueur allant de 0,05 mm à 6 mm.

Les particules non expansées utilisées dans la présente invention présentent généralement une masse volumique allant de 500 kg/m³ à 5000 kg/m³, en particulier de 900 kg/m³ à 3000 kg/m³, et plus particulièrement de 900 kg/m³ à 2000 kg/m³.

Les particules utilisées dans la présente invention peuvent être colorées ou incolores.

Comme particules utilisables dans les compositions de l'invention, on peut citer par exemple les microsphères non expansées de copolymère de chlorure de vinylidène/d'acrylonitrile/méthacrylate de méthyle comme par exemple celles commercialisées sous la dénomination d'« Expancel® » par la société AKZO NOBEL sous les références 820DU 40 (10-16 µm) ou 820 SL 40 (2-30 µm), et d'acrylonitrile/métahcrylate de méthyle comme par exemple celles commercialisées sous la dénomination d' « Expancel® » sous les références 642 WU 40 (10-16 µm) ou 051 DU 40 (9-15 µm). Comme particules pouvant être également utilisées dans les compositions selon l'invention, on peut encore citer les microsphères non expansées d'homopolymère d'acrylonitrile comme par exemple celles commercialisées sous la dénomination d'« Expancel 007W® » (5-25 µm), de « Micropearl F-series®» par la Société MATSUMOTO ou d'« Ucelite® » par la Société UCB.

Les particules commercialisées sous la dénomination d'«Expancel®» sous les références citées ci-dessus comprennent généralement dans leur cavité un composé à l'état gazeux.

Dans les compositions de l'invention, la teneur en composés susceptibles de gonfler sous l'action de la chaleur est généralement comprise entre 0,1 et 20%, et de préférence entre 0,2 et 15%, et encore plus préférentiellement entre 0,5 et 10% en poids du poids total de la composition.

Le milieu cosmétiquement acceptable est de préférence constitué par de l'eau ou par un ou plusieurs solvants cosmétiquement acceptables tels que des alcools ou par des mélanges eau-solvant(s). Ces solvants sont de préférence des alcools en C1-C4.

Parmi ces alcools, on peut citer l'éthanol, l'isopropanol, l'éthanol étant particulièrement préféré.

La composition selon l'invention peut contenir en outre au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques, amphotères ou zwitterioniques autres que les polymères fixants décrits ci-dessus, les huiles minérales, végétales ou synthétiques, les agents et tout autre additif classiquement utilisé dans les compositions cosmétiques, tels que les agents antipelliculaires, les agents anti-chute, les colorants, les pigments, les agents hydratants tels que la glycérine et les autres polyols, les réducteurs.

Ces additifs sont présents dans la composition à des teneurs avantageusement comprises entre 0,001 et 20% en poids du poids total de la composition. La quantité précise de chaque additif est fonction de sa nature et est déterminée facilement par l'homme du métier, et dépendra de l'application capillaire choisie.

Bien entendu, l'homme du métier veillera à choisir le ou les additifs de manière que les propriétés avantageuses de la composition conforme à l'invention ne soient pas, ou substantiellement pas, altérées par l'addition envisagée.

Les compositions de l'invention peuvent être utilisées pour la fabrication de nombreux produits capillaires, comme par exemple, des produits pour la fixation et/ou le maintien des cheveux, des produits de conditionnement, ou encore des produits de soin des cheveux.

Ces compositions peuvent être conditionnées sous diverses formes, notamment dans des flacons pompes ou dans des récipients aérosols, afin d'assurer une application de composition sous forme vaporisée ou sous forme de mousse. De telles formes de conditionnement sont indiquées, par exemple, lorsque l'on souhaite obtenir un spray, une laque, ou une mousse pour la fixation ou le traitement des cheveux. Les compositions conformes à l'invention peuvent également se présenter sous la forme de crèmes, de gels, d'émulsions, de lotions, ou de cires.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyléther.

Avantageusement, l'agent propulseur est présent à une concentration comprise entre 5 et 90% en poids par rapport au poids total au poids total de la composition dans le dispositif aérosol, et plus particulièrement à une concentration comprise entre 10 et 60%.

L'invention a également pour objet un procédé cosmétique de fixation et/ou de maintien de la coiffure, caractérisé en ce qu'il comprend l'application sur les cheveux d'une composition selon l'invention puis l'application de chaleur sur les cheveux, à une température comprise de préférence entre 55 et 220°C, et encore de préférence entre 65 et 220°C, pendant une durée pouvant être comprise entre 2 secondes et 30 minutes.

L'invention a enfin pour objet l'utilisation en cosmétique des compositions décrites ci-dessus. Elle a notamment pour objet l'utilisation de composés susceptibles de gonfler sous l'action de la chaleur pour la préparation de compositions de maintien et/ou de fixation de la coiffure.

L'exemple suivant est destiné à illustrer l'invention.

### Exemple 1 : utilisation capillaire de la composition selon l'invention

On applique la composition A suivante sur une mèche de cheveux naturels eurochâtain de 5 grammes.

### Composition A :

| | |
|---|---|
| Mexomère PW (1) | 6% MA |
| EXPANCEL 820 SLURRY (2) | 1% MA |
| Eau | 85g |
| Ethanol | 8g |
| MA : matières actives | |

(1) terpolymère acétate de vinyle/p-tertiobutyl benzoate de vinyle/acide crotonique neutralisé à 100% par le 2-amino-2-méthylpropanol-1.
(2) microsphères de vinylidène/acrylonitrile/méthacrylate de méthyle contenant de l'isobutane

La composition A est appliquée à l'aide d'un flacon pompe sur des cheveux naturels. La composition A permet d'obtenir une mise en forme et un maintien de la coiffure. Les cheveux sont ensuite soumis à l'action de la chaleur à l'aide d'un sèche-cheveux à 105°C pendant 20 secondes. La chaleur entraîne l'expansion non réversible des microsphères, ce qui fragilise les soudures entre les cheveux. Le coiffage et le démêlage des cheveux est alors aisé.

## Revendications

1. Procédé cosmétique de fixation et/ou de maintien de la coiffure, **caractérisé en ce qu'**on applique sur les cheveux une composition comprenant, dans un milieu cosmétiquement acceptable, au moins un polymère fixant et au moins un composé susceptible de gonfler sous l'action de la chaleur, puis **en ce qu'**on soumet les cheveux à l'action de la chaleur, à une température comprise entre 65 et 220°C.

2. Procédé selon la revendication 1, **caractérisé en ce que** le polymère fixant est choisi parmi les polymères fixants cationiques, anioniques, amphotères, non ioniques et leurs mélanges.

3. Procédé selon la revendication 2, **caractérisé en ce que** le polymère fixant cationique est choisi parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

4. Procédé selon la revendication 2, **caractérisé en ce que** le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

5. Procédé selon la revendication 2, **caractérisé en ce que** le polymère fixant amphotère est choisi parmi les copolymères à motifs vinyliques acides et à motifs vinyliques basiques, les polyaminoamides réticulés et acylés, les polymères à motifs zwittérioniques, les polymères dérivés du chitosane, les copolymères alkyl(C₁-C₅)vinyléther/anhydride maléique modifiés, les polyuréthanes amphotères et les polymères siliconés greffés amphotères.

6. Procédé selon la revendication 2, **caractérisé en ce que** le polymère fixant non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, les polyuréthanes non ioniques, et les polymères siliconés greffés non ioniques.

7. Procédé selon l'une quelconque des revendications précédentes, **caractérisé en ce que** le polymère fixant est présent dans la composition à des teneurs comprises entre 0,1 et 20%, de préférence entre 0,2 et 15%, et encore de préférence entre 0,5 et 10% en poids du poids total de la composition.

8. Procédé selon l'une quelconque des revendications précédentes, **caractérisé en ce que** le composé susceptible de gonfler sous l'action de la chaleur est sous la forme de particules thermoexpansibles.

9. Procédé selon la revendication 8, **caractérisé en ce que** lesdites particules sont capables de s'expanser sous l'action d'une température supérieure ou égale à 45°C, notamment à 50°C, en particulier à 60°C, plus particulièrement à 70°C.

10. Procédé selon la revendication 9, **caractérisé en ce que** ladite température est supérieure ou égale à 80°C, en particulier à 90°C et plus particulièrement à 100°C.

11. Procédé selon l'une quelconque des revendications 8 à 10, **caractérisé en ce que** les particules thermoexpansibles sont thermoplastiques.

12. Procédé selon l'une quelconque des revendications 8 à 11, **caractérisé en ce que** lesdites particules sont des particules creuses comportant une enveloppe continue et au moins une cavité.

13. Procédé selon la revendication 12, **caractérisé en ce que** ladite enveloppe comprend au moins un polymère résultant de la polymérisation de monomères choisis parmi les esters d'acides méthacrylique et acrylique, le chlorure de vinylidène, l'acrylonitrile, le styrène et ses dérivés, le butadiène et ses dérivés et leurs mélanges.

14. Procédé selon la revendication 13, **caractérisé en ce que** ledit polymère est choisi parmi les polymères comportant au moins des motifs dérivés d'acrylate ou de méthacrylate de méthyle, les polymères comportant au moins des motifs dérivés d'acrylonitrile, les polymères comportant au moins des motifs dérivés d'acrylonitrile et de méthacrylate de méthyle, les polymères comportant au moins des motifs dérivés de styrène et d'acrylonitrile, les polymères comportant au moins des motifs dérivés de chlorure de vinylidène et d'acrylonitrile, les polymères comportant au moins des motifs dérivés de chlorure de chlorure de vinylidène et de chlorure de vinyle.

15. Procédé selon la revendication 13 ou 14, **caractérisé en ce que** ledit polymère est choisi parmi les polymères de chlorure de vinylidène/acrylonitrile/méthacrylate de méthyle, les polymères d'acrylonitrile/méthacrylate de méthyle et les homopolymères d'acrylonitrile.

16. Procédé selon l'une quelconque des revendications 12 à 15, **caractérisé en ce que** ladite cavité contient au moins un composé capable de manifester, en réponse à un chauffage à une température située dans une gamme allant de 45°C à 200°C et à pression sensiblement constante, une augmentation d'au moins un facteur 30 de son volume par rapport à son volume à température ambiante.

17. Procédé selon la revendication 16, **caractérisé en ce que** ledit composé gazeux est choisi parmi l'air, l'azote, les hydrocarbures comprenant 1, 2, 3 ou 4 atomes de carbone et leurs mélanges.

18. Procédé selon la revendication 16 ou 17, **caractérisé en ce que** ledit composé est un composé liquide présentant une température de vaporisation située dans la gamme de 45°C à 200°C, en particulier dans la gamme de 80°C à 200°C.

19. Procédé selon la revendication 18, **caractérisé en ce que** ledit composé liquide est choisi parmi les hydrocarbures ayant de 5 à 15 atomes de carbone.

20. Procédé selon l'une quelconque des revendications 8 à 19, **caractérisé en ce que** les particules thermoexpansibles sont de forme globulaire ou allongées.

21. Procédé selon la revendication 20, **caractérisé en ce que** les particules thermoexpansibles sont sphériques et présentent une granulométrie, exprimée en diamètre « effectif » moyen en poids, allant de 0,5 µm à 200 µm, notamment de 1 µm à 100 µm, en particulier de 4 µm à 50 µm et plus particulièrement de 5 µm à 40 µm.

22. Procédé selon l'une quelconque des revendications 8 à 20, **caractérisé en ce que** les particules thermoexpansibles sont des fibres.

23. Procédé selon la revendication 22, **caractérisé en ce que** les fibres ont une longueur allant de 0,05 mm à 6 mm.

24. Procédé selon l'une quelconque des revendications 8 à 23, **caractérisé en ce que** les particules thermoexpansibles présentent une masse volumique allant de 500 kg/m³ à 5000 kg/m³, en particulier de 900 kg/m³ à 3000 kg/m³, et plus particulièrement de 900 kg/m³ à 2000 kg/m³.

25. Procédé selon l'une quelconque des revendications précédentes, **caractérisé en ce que** la teneur en composés susceptibles de gonfler sous l'action de la chaleur est comprise entre 0,1 et 20%, de préférence entre 0,2 et 15%, et encore de préférence entre 0,5 et 10% en poids du poids total de la composition.

26. Procédé selon l'une quelconque des revendications précédentes, **caractérisée en ce que** la composition comprend en outre au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques ou zwitterioniques autres que les polymères fixants figurant dans les revendications précédentes, les huiles minérales, végétales ou synthétiques, les agents et tout autre additif classiquement utilisé dans les compositions cosmétiques, les agents anti-pelliculaires, les agents anti-chute, les colorants, les pigments, les agents hydratants, les réducteurs.

27. Composition cosmétique capillaire, **caractérisée en ce qu'**elle comprend, dans un milieu cosmétiquement acceptable, au moins un polymère fixant et au moins un composé susceptible de gonfler sous l'action de la chaleur ayant une forme de fibres.

28. Composition selon la revendication 27, **caractérisée en ce que** les fibres ont une longueur allant de 0,05 mm à 6 mm.

29. Utilisation en cosmétique d'une composition selon la revendication 27 ou 28.

30. Utilisation de fibres susceptibles de gonfler sous l'action de la chaleur pour la préparation de compositions de maintien et/ou de fixation de la coiffure.
